# FASCICULE DE BREVET EUROPEEN

(11) **EP 3 518 018 B1**
(45) Date de publication et mention de la délivrance du brevet: **16.12.2020**
(21) Numéro de dépôt: 19154148.1
(22) Date de dépôt: 29.01.2019
(51) Int. Cl.: G02B 23/24, A61B 1/00, A61B 1/045, A61B 1/05

(54) **CAMÉRA D'ENDOSCOPIE INDUSTRIELLE ROTATIVE À TÊTE PIVOTANTE**
DREHBARE INDUSTRIELLE ENDOSKOPIEKAMERA MIT SCHWENKBAREM KOPF
ROTATING INDUSTRIAL ENDOSCOPY CAMERA WITH PIVOTING HEAD

(30) Priorité: 29.01.2018 FR 1850702
(43) Date de publication de la demande: 31.07.2019
(73) Titulaire: Foretec Societe Forezienne de Technologie, 42600 Montbrison (FR)
(72) Inventeur: ROCHE, Sylvain, 42600 Montbrison (FR)
(74) Mandataire: Weber, Jean-François

(56) Documents cités:
- EP-A1- 2 638 845
- WO-A1-2012/146664
- WO-A2-00/57770
- FR-A1- 3 017 217

## Description

La présente invention concerne le domaine de la prise de vue à distance, et en particulier celui des caméras utilisées en endoscopie industrielle, permettant notamment la visualisation de parois, d'objets ou de défauts inaccessibles au regard, et qui sont par exemple situés dans des cavités plus ou moins profondes et étroites, des tubes ou canalisations en particulier coudés, des forages, et/ou des zones dangereuses (zones radioactives, etc.).

La présente invention concerne plus précisément une caméra d'endoscopie industrielle comprenant au moins :
- un support destiné à être attaché à un organe de manoeuvre allongé du genre câble, jonc de poussée, perche, ou gaine souple ;
- un corps central embarquant un premier actionneur ;
- une structure de tête embarquant une unité de prise de vue et montée à rotation relativement audit corps central,
ledit premier actionneur étant relié à ladite structure de tête pour mettre en rotation cette dernière par rapport audit corps central.

On connaît une caméra d'endoscopie industrielle comprenant une unité de prise de vue, une structure de tête logeant l'unité de prise de vue, une structure intermédiaire rotative embarquant la structure de tête, et une structure de base interposée entre d'une part la structure intermédiaire rotative et d'autre part un jonc de poussée. La structure intermédiaire renferme un premier moteur qui contrôle le pivotement de la structure de tête par rapport à la structure intermédiaire, tandis que la structure de base renferme un second moteur contrôlant la rotation de la structure intermédiaire par rapport à la structure de base. Cette caméra connue permet ainsi d'effectuer des prises de vues selon un champ de vision orientable grâce au pivotement de la structure de tête et à la rotation de la structure intermédiaire, les images captées étant transmises à un moyen d'affichage distant par l'intermédiaire du jonc de poussée qui intègre un câble électrique.

Cette caméra d'endoscopie industrielle connue est de forme générale sensiblement cylindrique et présente des dimensions qui lui permettent d'être introduite à l'intérieur d'équipements comprenant des espaces cavitaires inaccessibles pour l'homme et à son regard, par exemple à l'intérieur de réseaux de canalisations ou de cuves. Le jonc de poussée relié à la caméra d'endoscopie permet à l'utilisateur de contrôler le cheminement de la caméra au sein de l'espace cavitaire, mais aussi de la récupérer une fois les prises de vues effectuées. Grâce à cette caméra, l'utilisateur peut ainsi contrôler l'état de l'intérieur d'un équipement ou d'un conduit, sans nécessairement avoir à y pénétrer lui-même ou à le démonter. Par ailleurs, dans le cas où un espace cavitaire contient une substance dangereuse pour la santé de l'utilisateur, par exemple une substance chimique ou une substance radioactive, il n'est pas nécessaire que celui-ci s'y expose physiquement, le contrôle visuel pouvant être effectué à distance à l'aide de la caméra d'endoscopie.

Cette caméra d'endoscopie industrielle connue donne globalement satisfaction, mais présente cependant certains inconvénients.

Cette caméra connue n'est ainsi pas adaptée pour passer dans certains espaces cavitaires relativement étroits et/ou sinueux, et en particulier des espaces cavitaires à la fois étroits et sinueux présentant notamment un faible diamètre et au moins un coude.

Par exemple, les différents modèles de cette caméra connue, y compris les modèles les plus compacts présentant un diamètre d'environ 45 mm, ne sont pas capables de circuler à l'intérieur d'un conduit présentant d'une part un diamètre nominal 50 ou DN50, c'est-à-dire notamment une section transversale de diamètre extérieur ou intérieur d'environ 50 mm, et d'autre part un (ou plusieurs) coude(s) à 90°, c'est-à-dire à angle droit. Or, il existe en pratique un réel besoin d'inspecter de tels conduits, voire des conduits encore plus étroits et/ou encore plus sinueux (c'est-à-dire présentant un ou plusieurs angles à plus de 90°, par exemple 120°) dans diverses installations industrielles.

On connait cependant d'autres caméras d'endoscopie de petites dimensions qui sont capables de cheminer au sein d'un conduit relativement étroit et sinueux tel que susmentionné, mais leur robustesse, leur qualité d'image, leur masse, leur étanchéité et le contrôle de l'orientation de leur champ de vision ne sont pas pleinement satisfaisants en pratique.

Le document FR-3 017 217 décrit un dispositif de télé-visualisation comprenant une sonde vidéo incluant une unité de prise de vue, un organe de manœuvre allongé pour manœuvrer la sonde, et un système de contrôle à distance de la sonde vidéo.

Les objets assignés à la présente invention visent en conséquence à remédier aux différents inconvénients énumérés précédemment et à proposer une nouvelle caméra d'endoscopie industrielle dont la conception permet de lui conférer, tout en préservant sa maniabilité et sa fiabilité, un caractère particulièrement compact, avec un excellent compromis longueur/diamètre, et une masse limitée.

Un autre objet de l'invention vise à proposer une nouvelle caméra d'endoscopie industrielle de conception particulièrement robuste.

Un autre objet de l'invention vise à proposer une nouvelle caméra d'endoscopie industrielle dont la conception lui confère une relative légèreté.

Un autre objet de l'invention vise à proposer une nouvelle caméra d'endoscopie industrielle de conception à la fois peu encombrante et de taille suffisante pour loger une unité de prise de vue garantissant une bonne qualité d'image, et en particulier une bonne qualité d'image vidéo.

Un autre objet de l'invention vise à proposer une nouvelle caméra d'endoscopie industrielle dont la fabrication est facile à mettre en œuvre et à coût maîtrisé.

Un autre objet de l'invention vise à proposer une nouvelle caméra d'endoscopie industrielle nécessitant une maintenance minimale.

Un autre objet de l'invention vise à proposer une nouvelle caméra d'endoscopie industrielle polyvalente, permettant notamment d'accéder à des cavités relativement étroites, par exemple l'intérieur d'un tube ou d'un équipement, en particulier une cavité présentant un diamètre réduit et/ou un coude.

Un autre objet de l'invention vise à proposer une nouvelle caméra d'endoscopie industrielle présentant une bonne étanchéité y compris dans le temps.

Un autre objet assigné à l'invention vise à proposer une nouvelle caméra d'endoscopie industrielle de construction particulièrement simple et dont la conception permet de maintenir le diamètre de la caméra, et plus spécialement le diamètre d'une structure de tête ou boule de visualisation de la caméra, relativement réduit, et notamment en deçà de 30 mm, et ce quelle que soit la direction de visualisation de la caméra.

Un autre objet assigné à l'invention vise à proposer une nouvelle caméra d'endoscopie industrielle qui est facile et rapide à monter ou à démonter sur un organe de manœuvre allongé du genre câble, jonc de poussée, perche, ou gaine souple, et à manœuvrer à l'aide de cet organe, et ce même dans une large variété d'espaces cavitaires.

Les objets assignés à l'invention sont atteints à l'aide d'une caméra d'endoscopie industrielle comprenant au moins :
- un support destiné à être attaché à un organe de manœuvre allongé du genre câble, jonc de poussée, perche, ou gaine souple ;
- un corps central embarquant un premier actionneur ;
- une structure de tête embarquant une unité de prise de vue et montée à rotation relativement audit corps central,
ledit premier actionneur étant relié à ladite structure de tête pour mettre en rotation cette dernière par rapport audit corps central, le corps central étant monté à rotation relativement audit support, caractérisée en ce que le corps central embarque en outre un second actionneur destiné à mettre en rotation ledit corps central par rapport audit support, ledit premier actionneur et/ou ledit second actionneur étant positionné(s) sensiblement à l'extérieur dudit support.

D'autres objets et avantages de l'invention ressortiront plus en détails à la lecture de la description qui suit, et à l'aide des figures annexées fournies à titre purement explicatif et non limitatif, dans lesquels :
- La figure 1 illustre, selon une vue de côté, une caméra d'endoscopie industrielle selon l'invention.
- La figure 2 illustre, selon une vue de face, la caméra d'endoscopie industrielle représentée à la figure 1.
- La figure 3 illustre la caméra d'endoscopie industrielle des figures 1 et 2 selon une vue en coupe sagittale selon le plan de coupe BB (figure 1).
- La figure 4 illustre, selon une vue en perspective, la caméra d'endoscopie industrielle des figures précédentes dépourvues toutefois de certaines pièces (boîtiers de protection).
- La figure 5 illustre, selon une vue en perspective, un détail de la partie supérieure de la caméra d'endoscopie industrielle représentée à la figure 4.
- La figure 6 illustre, selon une vue en perspective, un détail de la partie inférieure de la caméra d'endoscopie industrielle représentée à la figure 4, dépourvue de certaines pièces (boîtiers de protection).
- La figure 7 est un schéma fonctionnel de la caméra d'endoscopie représentée aux figures précédentes.
- La figure 8 illustre, selon une vue de côté, une partie d'un organe de manœuvre auquel la caméra d'endoscopie industrielle des figures précédentes est destinée à être attachée.

L'invention concerne une caméra d'endoscopie industrielle 1. La caméra d'endoscopie industrielle 1 selon l'invention permet à un utilisateur, lorsqu'elle est reliée à un moyen d'affichage approprié, de visualiser, en particulier à distance, l'image d'un objet. La caméra d'endoscopie industrielle 1 permet en particulier d'observer un objet situé dans un espace creux et/ou inaccessible pour l'utilisateur, l'espace creux étant ainsi par exemple délimité par les parois intérieures d'un tube, d'un conduit ou d'une canalisation présentant optionnellement des coudes ou des chicanes, le tube, le conduit ou la canalisation présentant notamment une section sensiblement circulaire ou elliptique. L'espace creux peut également se présenter simplement comme une cavité interne d'un équipement. Bien entendu, l'espace creux pourra correspondre à tout espace intérieur d'une machine, d'un bâtiment ou d'une installation. La caméra d'endoscopie industrielle 1 permet ainsi avantageusement d'explorer visuellement l'espace creux, et en particulier d'examiner visuellement, de l'intérieur, l'état des bords ou parois délimitant l'espace creux, ou encore l'état d'un dispositif placé à l'intérieur dudit espace creux, par exemple une pompe, une grille, une turbine, etc. La caméra d'endoscopie industrielle 1 permet en particulier, notamment lorsqu'elle est reliée audit moyen d'affichage, de mettre en oeuvre une méthode non destructive de contrôle visuel indirect de l'espace creux, c'est-à-dire qui ne demande de préférence pas la destruction ou la détérioration du tube, du conduit, de la canalisation, de l'équipement ou de la machine à l'intérieur duquel ou de laquelle se situe l'espace creux.

La caméra d'endoscopie industrielle 1 est particulièrement adaptée pour l'inspection visuelle à distance de l'intérieur d'un tuyau, tube ou conduit de faible diamètre, par exemple un diamètre intérieur de 50 mm et/ou un diamètre nominal DN50, et qui présente un ou plusieurs coude(s), par exemple un coude à angle droit, c'est-à-dire formant une courbure à 90° environ.

La caméra d'endoscopie industrielle 1 présente préférentiellement une forme et des dimensions adaptées à son utilisation. Par exemple, elle présente une forme générale sensiblement allongée, fuselée, prismatique, cylindrique et/ou cylindro-conique, et une longueur, une largeur, une épaisseur et/ou un diamètre adaptés, c'est-à-dire de grandeur suffisante pour assurer les différentes fonctions de la caméra 1 tout en pouvant évoluer dans un espace creux (en particulier d'un conduit, d'une machine, etc.).

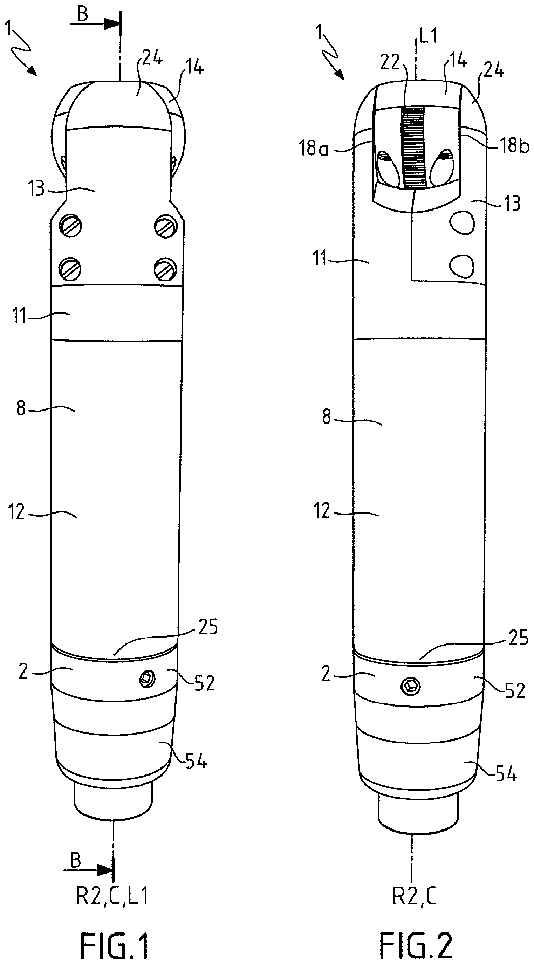

La caméra d'endoscopie industrielle 1 forme de préférence une caméra d'inspection vidéo (comme illustré aux figures 1 à 5) d'un espace creux situé par exemple à l'intérieur d'une cuve ou d'une canalisation d'un réseau de canalisations hydrauliques, aérauliques, électriques etc. Bien entendu, la caméra d'endoscopie industrielle 1 pourra également être utilisée pour observer des espaces ouverts, en particulier lorsqu'ils sont difficiles d'accès pour l'utilisateur, par exemple un lieu escarpé, ou dangereux pour l'utilisateur, c'est-à-dire par exemple situés à proximité d'éléments nocifs à la santé dudit utilisateur, tels que des substances radioactives ou toxiques, ou encore des explosifs ou des particules fines ou pulvérulentes.

Le caméra d'endoscopie industrielle peut également permettre à l'utilisateur d'observer un espace creux dont la température interne est très élevée, comme par exemple l'intérieur d'une enceinte de combustion d'une centrale thermique, à l'intérieur de laquelle règne une température qui peut par exemple être supérieure à 1800°C.

Selon l'invention, et comme illustré aux figures 1 à 4 et 6, la caméra d'endoscopie industrielle 1 comprend au moins un support 2, lequel sert avantageusement de structure de référence, ou bâti, pour les autres parties de la caméra d'endoscopie industrielle 1 qui seront décrites ci-après. De manière avantageuse, ledit support 2 présente une forme générale de révolution par exemple sensiblement cylindrique.

Selon l'invention également, ledit support 2 est destiné à être attaché à un organe de manœuvre 3 allongé du genre câble, jonc de poussée, perche, ou gaine souple. En d'autres termes, ledit support 2 est avantageusement conçu pour qu'un utilisateur puisse déplacer ladite caméra d'endoscopie industrielle 1 dans un espace creux à inspecter qui peut notamment être étroit et/ou coudé, et ce à l'aide dudit organe de manoeuvre 3, notamment en poussant ou en tirant ce dernier vis-à-vis dudit espace creux (ou cavité). Ainsi, ledit support 2 permet, de manière avantageuse, de manipuler l'ensemble de la caméra d'endoscopie industrielle 1 via ledit organe de manœuvre 3, lequel est par exemple destiné à être rattaché à une base de la forme de révolution (et de préférence cylindrique) dudit support 2.

Bien évidemment, il est tout à fait envisageable que la caméra d'endoscopie 1 de l'invention comprenne en outre ledit organe de manœuvre 3 allongé du genre câble, jonc de poussée, perche, ou gaine souple.

Ledit organe de manœuvre 3 peut donc être formé principalement par une variété d'objets allongés appropriés pour guider la caméra d'endoscopie industrielle 1 de préférence rattachée à une extrémité dudit objet allongé. L'organe de manœuvre 3 comprend avantageusement au moins un connecteur (non illustré) destiné à être relié audit support 2, ledit connecteur assurant idéalement une liaison (par exemple électrique, vidéo, etc.) entre la caméra d'endoscopie industrielle 1 et l'extérieur de l'espace creux à inspecter. Ledit organe de manœuvre 3 est de préférence destiné à assurer une liaison mécanique, électrique et vidéo avec l'extérieur d'un espace creux à inspecter, permettant ainsi de contrôler la progression (c'est-à-dire le déplacement, ou la position) de la caméra d'endoscopie industrielle 1 dans l'espace creux en tirant ou en poussant sur un câble, un jonc de poussée, une perche, ou une gaine souple formant (principalement) ledit organe de manœuvre 3.

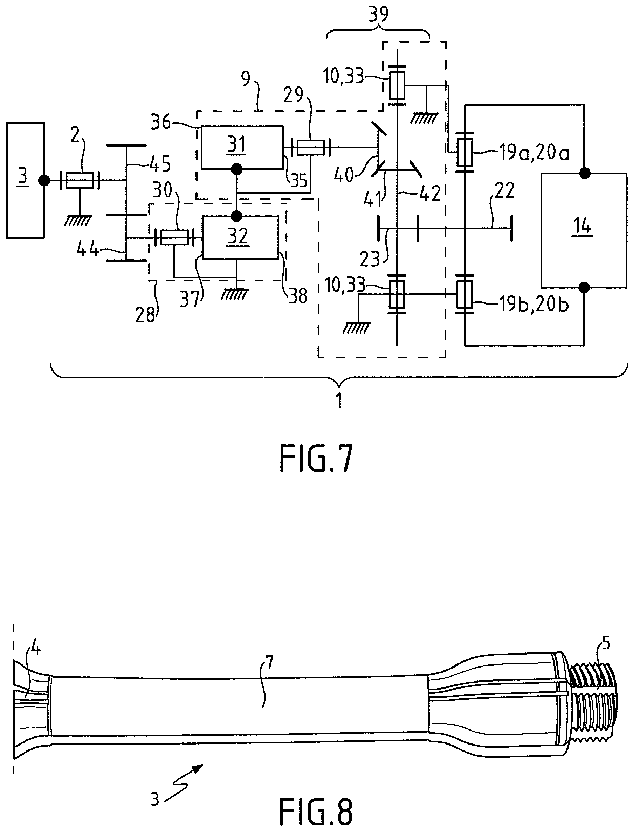

De manière avantageuse, ledit organe de manoeuvre 3 est au moins partiellement flexible et comprend, comme illustré à la figure 8, au moins une partie flexible 7, grâce à laquelle, seule ou en combinaison avec d'autres parties flexibles dudit organe de manœuvre 3, ce dernier peut faire passer la caméra d'endoscopie industrielle 1 dans des éventuel(le)s coudes ou courbures d'un espace creux à inspecter. Alternativement, l'organe de manœuvre 3 est (au moins en partie, voire en totalité) sensiblement rigide, par exemple il forme principalement une perche métallique peu ou non flexible. De préférence, l'organe de manœuvre 3 est semi-rigide, c'est-à-dire qu'il comprend au moins ladite partie flexible 7 et une partie rigide, et présente hors utilisation une forme générale sensiblement allongée, par exemple une forme générale de barre ou de tuyau, ou bien alternativement un aspect semblable à celui d'une corde, d'un câble (par exemple il est au moins partiellement enroulé) ou d'une gaine (par exemple destinée à abriter des fils électrique) de préférence souple. De préférence, l'organe de manœuvre 3 comprend un premier et un second bouts 4, 5, le premier bout 4 étant pourvu du connecteur de l'organe de manœuvre 3 et donc destiné à être relié à la caméra d'endoscopie industrielle 1, le second bout 5 étant destiné à être manipulé par l'utilisateur et/ou relié à un autre équipement de type câble, jonc de poussée, perche, ou gaine souple.

Selon l'invention, et comme illustré aux figures 1 à 3, la caméra d'endoscopie industrielle 1 comprend un corps central 8 embarquant au moins un premier actionneur 9. Ainsi, la caméra d'endoscopie industrielle 1 comprend de manière avantageuse un corps central 8 et un premier actionneur 9, ce dernier étant par exemple monté (c'est-à-dire installé et/ou positionné) au moins en partie (voire en totalité) dans ledit corps central 8 (c'est-à-dire au sein / à l'intérieur de ce dernier). En d'autres termes, le corps central 8 supporte avantageusement en son sein le premier actionneur 9 (au moins en partie voire en totalité).

Avantageusement, et comme illustré aux figures 1 à 3, ledit corps central 8 présente une forme sensiblement allongée s'étendant longitudinalement entre une extrémité primaire 24 et une extrémité secondaire 25, de préférence selon une première direction d'extension longitudinale L. De façon avantageuse, ledit corps central 8 présente au moins en partie une forme élancée (c'est-à-dire allongée, par exemple selon ladite première direction d'extension longitudinale L), fuselée, prismatique, cylindrique et/ou cylindro-conique.

Avantageusement, ledit support 2 est conçu pour que ledit organe de manœuvre 3 puisse être lui être rattaché sensiblement à l'opposé dudit corps central 8, comme illustré aux figures. Par exemple, comme illustré aux figures, le support 2 s'étend entre une première et une seconde partie extrême opposées, ledit corps central 8 étant relié audit support 2 au niveau de ladite première partie extrême, tandis que ledit organe de manoeuvre 3 est destiné à venir se fixer audit support 2 au niveau de ladite seconde partie extrême, lesdits organe de manoeuvre 3 et corps central 8 étant donc positionnés à l'opposé l'un de l'autre par rapport audit support 2. De préférence, ledit corps central 8 présente une forme sensiblement allongée dans le prolongement de laquelle se situe la majorité et avantageusement la quasi-totalité ou la totalité du support 2, lequel présente optionnellement également une forme sensiblement allongée. De façon avantageuse, lesdits corps central 8 et support 2 constituent un sous-ensemble de forme générale sensiblement allongée dans le prolongement de laquelle s'étend au moins une partie de l'organe de manœuvre 3, par exemple ledit premier bout 4, qui est donc de préférence fixé à l'opposé du corps central 8 par rapport au support 2. Par exemple, le premier bout 4 de l'organe de manœuvre 3 est destiné à être rattaché au support 2 au niveau de la seconde partie extrême de ce dernier, tandis que l'extrémité secondaire 25 du corps central 8 est reliée au support 2 au niveau de la première partie extrême de ce dernier.

Ledit premier actionneur 9 est en particulier positionné au moins en partie à l'intérieur du corps central 8, notamment de façon à être solidaire à ce dernier mais de manière réversible. Pour faciliter la réalisation du corps central 8, et le montage du premier actionneur 9 dans ledit corps central 8, ce dernier comprend avantageusement plusieurs sous-parties qui sont assemblées les unes aux autres par exemple par soudage ou vissage, et notamment une première, une deuxième et une troisième sous-partie 11, 12, 13, comme illustré aux figures, lesdites première, une deuxième et une troisième sous-partie 11, 12, 13 étant préférentiellement complémentaires les unes des autres pour former le corps central 8.

Toujours selon l'invention, et comme illustré aux figures 1 à 5, la caméra d'endoscopie industrielle 1 comprend en outre une structure de tête 14. De préférence, ledit corps central 8 embarque ladite structure de tête 14, laquelle est ainsi avantageusement positionnée au moins en partie à l'intérieur dudit corps central 8, et encore plus précisément au moins en partie entre lesdites première et troisième sous-parties 11, 13. Par exemple, ledit corps central 8 comprend, à ladite extrémité primaire 24, une première cavité 26, ladite structure de tête 14 étant logée au moins en partie dans ladite première cavité 26, et il comprend en outre, à l'extrémité secondaire 25, une seconde cavité 27, ledit support 2 étant logé au moins en partie dans ladite seconde cavité 27. De préférence, ladite seconde cavité 27 s'étend à l'intérieur dudit corps central 8, au voisinage immédiat de ladite extrémité secondaire 25, par exemple entre cette dernière et un point situé à l'intérieur du corps central 8 et à distance de ladite extrémité primaire 24. De manière préférentielle également, ladite première cavité 26 s'étend à l'intérieur dudit corps centra! 8, au voisinage immédiat de ladite extrémité primaire 24. Ainsi, ledit support 2 se trouve avantageusement positionné sensiblement à l'opposé de ladite structure de tête 14 par rapport audit corps central 8.

De manière avantageuse, ladite structure de tête 14, ledit corps central 8, ledit support 2 et ledit organe de manœuvre 3 sont sensiblement positionnés les uns derrière les autres successivement dans cet ordre, comme illustré aux figures. Ledit organe de manœuvre 3 et ladite structure de tête 14 sont donc avantageusement positionnés à l'opposé l'un de l'autre par rapport auxdits support 2 et corps central 8, lesdits support 2 et corps central 8 étant ainsi préférentiellement positionnés entre lesdits organe de manœuvre 3 et structure de tête 14. Par exemple, ledit organe de manœuvre 3 est destiné à venir partiellement s'emboîter dans ledit support 2 ou inversement, ledit support 2 étant destiné à venir s'emboîter (de manière mobile, plus précisément monté à rotation) partiellement au sein dudit corps central 8, lequel embarque ladite structure de tête 14 qui se retrouve donc en partie et de préférence en totalité « *emboîtée »* au sein de ce dernier (de manière mobile, plus précisément montée à rotation). De façon préférentielle, ladite structure de tête 14, ledit corps central 8, ledit support 2 et ledit organe de manœuvre 3 sont tous des éléments distincts les uns des autres.

De façon avantageuse, la caméra d'endoscopie 1 est destinée à inspecter un espace creux d'une installation industrielle pourvu en particulier d'un ou plusieurs coudes. La caméra d'endoscopie industrielle 1 est de préférence conçue pour être introduite toute entière au sein d'un espace creux à inspecter, à l'exception éventuellement d'une partie de l'organe de manœuvre 3 (lorsqu'il fait partie de la caméra 1) manié à un bout non introduit par un utilisateur. Ainsi, la caméra 1 est avantageusement conçue (c'est-à-dire notamment dimensionnée) pour que ladite structure de tête 14, ledit corps central 8, ledit support 2, et au moins une partie de l'organe de manoeuvre 3 puissent être introduits au sein d'un espace creux à inspecter, lequel est par exemple muni d'au moins un coude. De préférence, lesdits premier et second actionneurs 9, 28, et préférentiellement lesdits premier et second moteurs 31, 32, sont, eux aussi, destinés à être entièrement introduits au sein d'un espace creux à inspecter, avec le reste de la caméra 1 .

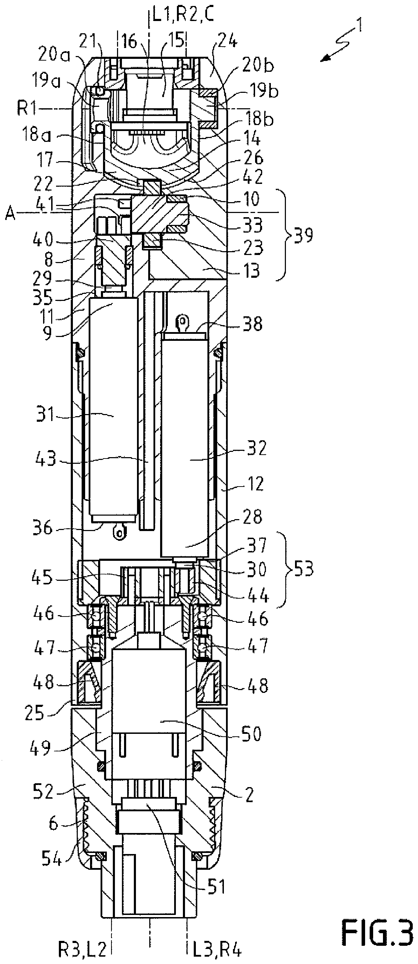

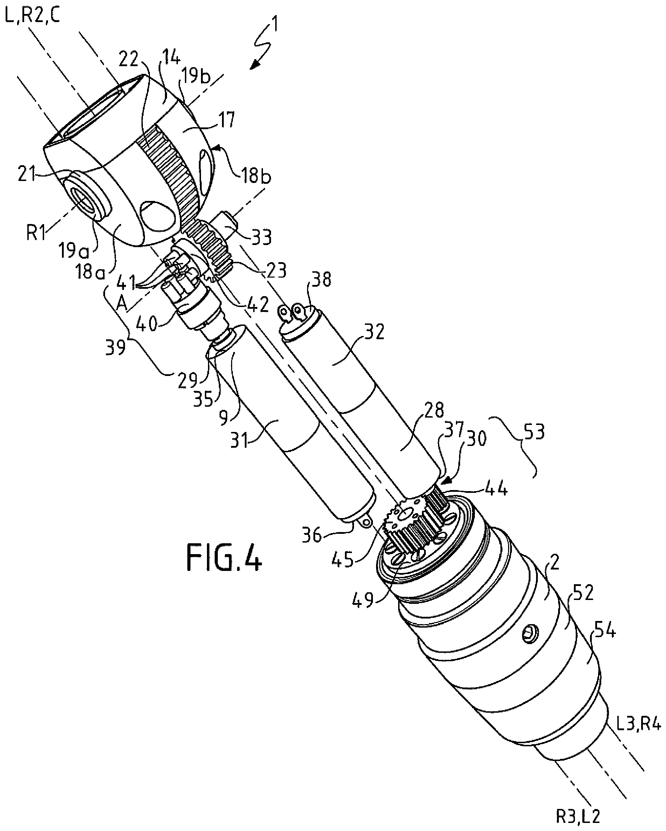

Selon l'invention, ladite structure de tête 14 embarque une unité de prise de vue 15, comme illustré aux figures 3 et 4. Ainsi, la caméra d'endoscopie industrielle 1 comprend en particulier une unité de prise de vue 15 embarquée par la structure de tête 14. En d'autres termes, l'unité de prise de vue 15 est de préférence au moins en partie, voire en totalité, montée sur la structure de tête 14 ou à l'intérieur de celle-ci, ou encore intégrée à la structure de tête ou à l'intérieur de celle-ci. Par exemple, ladite structure de tête 14 présente une section avec une forme en U, et comprend un espace interne 16 correspondant au creux de ladite forme en U, dans lequel est logé (c'est-à-dire monté ou embarqué) au moins en partie ladite unité de prise de vue 15.

L'unité de prise de vue 15 est ainsi avantageusement conçue pour capter des images de l'environnement de la caméra d'endoscopie industrielle 1, et comprend en particulier une caméra vidéo ou un appareil photo embarqué(e). En d'autre termes, ladite unité de prise de vue 15 permet, de manière avantageuse, d'effectuer une prise de vue, c'est-à-dire en particulier de capturer sur un support de prise de vue (par exemple un support numérique) d'une image ou d'une vidéo d'un sujet (par exemple un objet, une paroi à l'intérieur d'un tuyau, etc.). Préférentiellement, l'unité de prise de vue 15 comprend un capteur, par exemple un capteur de température, de distance, de position et/ou d'horizontalité, et un système de mise au point de la prise de vue, ce dernier comprenant lui-même en particulier une lentille liquide pilotable à distance. Comme sur une caméra classique, l'unité de prise de vue 15 présente de préférence un champ de vision et permet avantageusement d'obtenir une image sensiblement nette d'un objet optique situé dans son champ de vision, par réglage du système de mise au point. De préférence, il est possible pour l'utilisateur de sélectionner l'objet optique qu'il désire voir net, selon que l'objet en question est placé plutôt au premier plan ou au dernier plan. Le moyen de mise au point peut être à réglage automatique (« *autofocus* ») et/ou manuel.

L'unité de prise de vue 15 comprend de préférence également un moyen d'éclairage de son champ de vision. En effet, le moyen d'éclairage peut se révéler particulièrement utile dans le cas où l'espace creux à inspecter présente une luminosité naturelle trop faible pour être perçue par l'unité de prise de vue 15, ou présente une luminosité nulle.

Selon l'invention également, ladite structure de tête 14 est montée à rotation relativement audit corps central 8 de préférence selon un premier axe de rotation R1, lequel est avantageusement sensiblement perpendiculaire à ladite première direction d'extension longitudinale L. Ainsi, ladite structure de tête 14 est de préférence reliée directement (ou alternativement indirectement) au corps central 8, et peut effectuer un mouvement de rotation par rapport audit corps central 8 selon ledit premier axe de rotation R1. De préférence, ladite structure de tête 14 comme l'unité de prise de vue 15 se trouvent dans le prolongement dudit corps central 8 (et plus précisément au sein de ce dernier) et sont traversées par ladite première direction d'extension longitudinale L.

Par exemple, comme illustré aux figures 1 à 5, ladite structure de tête 14 comprend une demi-coquille comprenant elle-même une paroi sensiblement incurvée 17 (correspondant en particulier à la base de la forme en U), deux parois planes 18a, 18b (correspondant en particulier auxdites deux ailes de la forme en U) opposées sensiblement parallèles et reliées par ladite paroi incurvée 17, deux parties d'arbre 19a, 19b situées sur l'extérieur desdites deux parois planes 18a, 18b et par lesquelles passe le premier axe de rotation R1. Ledit corps central 8 comprend quant à lui avantageusement deux paliers 20a, 20b (ou logements) chacun conçu pour recevoir l'une correspondante desdites deux parties d'arbre 19a, 19b et avantageusement pratiqués chacun dans l'une différentes desdites première et troisième sous-parties 11, 13, l'interface palier 20a, 20b / partie d'arbre 19a, 19b correspondante permettant ainsi le pivotement (c'est-à-dire la mise en rotation) de ladite structure de tête 14 par rapport audit corps central 8 selon le premier axe de rotation R1. La caméra d'endoscopie industrielle 1 comprend également, de manière avantageuse et comme illustré à la figure 3, un premier joint 21, par exemple formé par un joint torique, positionné entre dans un des paliers 20a et/ou autour d'une des parties d'arbre 19a afin d'assurer l'étanchéité du corps central 8. Ainsi, de préférence, le corps central 8 embarque ladite structure de tête 14, celle-ci se trouvant avantageusement montée au moins en partie dans le corps central 8 par exemple entre lesdites première et troisième sous-parties 11, 13.

Toujours selon l'invention, ledit premier actionneur 9 est relié à ladite structure de tête 14 pour mettre en rotation cette dernière par rapport audit corps central 8. Le premier actionneur 9 est ainsi avantageusement relié d'une part au corps central 8 et d'autre part à la structure de tête 14, afin de faire réaliser à cette dernière un déplacement en rotation par rapport audit corps central 8. Ainsi, la mise en marche dudit premier actionneur 9 entraîne avantageusement un mouvement de rotation (ou pivotement) selon le premier axe de rotation R1 de ladite structure de tête 14. De façon avantageuse, le premier axe de rotation R1 passe sensiblement horizontalement vis-à-vis ladite forme en U, de manière à être sensiblement perpendiculaire aux ailes et parallèle à la base de ladite forme en U. De manière préférentielle, ledit espace interne 16 s'étend sensiblement de part et d'autre d'un plan moyen passant par ladite première direction d'extension longitudinale L.

Par exemple, ladite paroi incurvée 17 comprend sur une face extérieure une série d'évidements 22 (ou alternativement une série d'excroissances). Le premier actionneur 9 comprend quant à lui avantageusement une roue dentée primaire 23 (et plus précisément ses dents) sensiblement complémentaire desdites évidements 22, c'est-à-dire conçue pour former un système d'engrenage avec ceux-ci, positionnée en correspondance avec ces derniers. Ladite roue dentée primaire 23 est de préférence positionnée au moins en partie au sein (c'est-à-dire à l'intérieur) dudit corps central 8, et montée à rotation relativement audit corps central 8 selon un axe de rotation primaire A lequel est avantageusement sensiblement parallèle audit premier axe de rotation R1 et/ou perpendiculaire à ladite première direction d'extension longitudinale L. Ainsi, de façon avantageuse, le mouvement de rotation de ladite roue dentée primaire 23 selon l'axe de rotation primaire A entraîne le parcours de la roue dentée primaire 23 dans les évidements 22 (et donc le long de la paroi incurvée 17) et donc la rotation de la structure de tête 14 selon le premier axe de rotation R1 grâce au premier système de roulement 21 en combinaison avec les deux paliers 20a, 20b et les deux parties d'arbre 19a, 19b.

Selon une caractéristique de l'invention, comme illustré aux figures 1 à 3, le corps central 8 est monté à rotation relativement audit support 2, de préférence selon un deuxième axe de rotation R2, lequel est avantageusement sensiblement perpendiculaire audit premier axe de rotation R1 et/ou sensiblement parallèle à ou confondu avec ladite première direction d'extension longitudinale L. Ainsi, ledit corps central 8 est de préférence relié au support 2, et peut effectuer un mouvement de rotation relativement audit support 2 selon ledit deuxième axe de rotation R2. Préférentiellement, ledit support 2 se trouve dans le prolongement dudit corps central 8 et est traversé par ladite première direction d'extension longitudinale L. Le corps central 8 peut avantageusement effectuer une rotation à au moins 180°, voire au moins 360°, et ce dans le(s) sens horaire et/ou antihoraire relativement audit support 2. De façon avantageuse, ledit corps central 8 et ledit support 2, lorsqu'ils sont reliés ensemble, forment un sous-ensemble sensiblement allongé, s'étendant par exemple entre lesdites extrémité primaire 24 et seconde partie extrême, et présentant une section transversale sensiblement constante, c'est-à-dire variant peu (par exemple variant de moins de 10%) sur la majorité de la longueur dudit sous-ensemble, par exemple au moins 80%, de préférence au moins 90% voire la totalité de la longueur dudit sous-ensemble. Cette configuration présentant une certaine continuité de forme est particulièrement avantageuse dans le cadre de l'invention, puisque la caméra d'endoscopie 1 industrielle est préférentiellement destinée à évoluer au sein d'un espace creux à inspecter notamment pourvu d'un ou plusieurs coudes, sa forme cohérente lui permettant d'évoluer toute entière au sein dudit espace creux et plus particulièrement dudit coude sans difficulté.

Ainsi, pour visualiser un objet se trouvant dans un espace creux à inspecter avec la nouvelle caméra d'endoscopie industrielle 1, ledit objet se trouvant au voisinage de la caméra d'endoscopie industrielle 1, on peut réaliser, de manière avantageuse, une rotation de la structure de tête 14 selon le premier axe de rotation R1 et une rotation du corps central 8 selon le deuxième axe de rotation R2, ce qui permet d'ajuster le champ de vision de l'unité de prise de vue 15 à l'objet à visualiser, ou en d'autres termes de mettre l'objet dans le champ de vision de l'unité de prise de vue 15 en particulier lorsque cette dernière (et surtout son champ de vision) n'est pas déjà tournée vers ledit objet.

Selon une autre caractéristique de l'invention, le corps central 8 embarque en outre un second actionneur 28 destiné à mettre en rotation ledit corps central 8 par rapport audit support 2. Ainsi, la caméra d'endoscopie industrielle 1 comprend également un second actionneur 28, ce dernier étant par exemple monté (c'est-à-dire installé et/ou positionné) au moins en partie (voire en totalité) dans ledit corps central 8 (c'est-à-dire au sein / à l'intérieur de ce dernier). En d'autres termes, le corps central 8 supporte avantageusement en son sein le second actionneur 28 (au moins en partie voire en totalité). Le second actionneur 28 est donc avantageusement relié d'une part au support 2 et d'autre part au corps central 8, afin de faire réaliser à ce dernier un déplacement en rotation par rapport au support 2.

La caméra d'endoscopie industrielle 1 comprend ainsi un premier et un second actionneur 9, 28, tous deux étant préférentiellement embarqués par ledit corps central 8. En particulier, lorsque le second actionneur 28 est mis en marche, le corps central 8, ainsi que la structure de tête 14 et l'unité de prise de vue 15 (par exemple montées sur et/ou dans le corps central 8, et/ou embarquées par celui-ci), se déplacent de manière avantageuse en rotation par rapport au support 2 selon le deuxième axe de rotation R2. En d'autres termes, la mise en marche dudit second actionneur 28 implique avantageusement un déplacement rotatif, par rapport au support 2 et selon le deuxième axe de rotation R2, desdits premier et second actionneurs 9, 28 embarqués par le corps central 8.

De manière préférentielle, lesdits premier et second actionneurs 9, 28 sont positionnés (au moins en partie, voire en totalité) à l'intérieur du corps central 8 entre lesdites première et seconde cavités 26, 27.

Selon l'invention, ledit premier actionneur 9 et/ou ledit second actionneur 28 est/sont positionné(s) sensiblement à l'extérieur dudit support 2, comme cela est illustré aux figures. De préférence, les deux actionneurs 9, 28, c'est-à-dire lesdits premier et second actionneurs 9, 28, sont positionnés au moins en partie à l'extérieur dudit support 2 et même totalement en dehors de ce dernier. En d'autres termes, au moins l'un et de préférence les deux desdits premier et second actionneurs 9, 28 est (/sont) positionné(s) principalement, préférentiellement en totalité, en dehors du support 2.

De façon avantageuse, lesdits premier et second actionneurs 9, 28 comprennent respectivement un premier et un deuxième arbre rotatif 29, 30, lesdits premier et deuxième arbres rotatifs 29, 30 étant sensiblement parallèles (et avantageusement sensiblement allongés).

Préférentiellement, comme illustré aux figures 3 à 6, lesdits premier et second actionneurs 9, 28 comprennent respectivement un premier et un second moteur 31, 32 pour mettre en rotation respectivement lesdits premier et deuxième arbres rotatifs 29, 30. De manière encore plus préférentielle, ledit premier moteur 9 est principalement formé par un premier moteur électrique, et ledit second moteur 28 est principalement formé par un second moteur électrique. Ainsi, chacun desdits premier et second moteurs 9, 28 est avantageusement formé par un moteur électrique distinct.

De façon avantageuse, comme illustré aux figures 3 à 6, lesdits premier et second moteurs 31, 32 présentent chacun une forme sensiblement allongée et sont disposés sensiblement parallèlement l'un à l'autre au sein du corps central 8. En d'autres termes, lesdits premier et second moteurs 31, 32 présentent avantageusement chacun une forme sensiblement allongée s'étendant longitudinalement selon respectivement une deuxième et une troisième direction d'extension longitudinale L2, L3. Plus avantageusement encore, lesdites deuxième et troisième directions d'extension longitudinales L2, L3 sont sensiblement parallèles l'une à l'autre ou confondues l'une avec l'autre. Lesdits premier et second moteurs 31, 32 sont ainsi avantageusement positionnés à l'intérieur du corps central 8 côte à côte et parallèlement l'un à l'autre.

Une telle configuration permet notamment d'obtenir une caméra d'endoscopie industrielle 1 présentant une dimension latérale ou radiale sensiblement réduite, les premier et second moteurs 31, 32 étant avantageusement positionnés sensiblement parallèlement à la première direction d'extension longitudinale L1 du corps central 8. En d'autres termes, il est possible, grâce à cette configuration, de mettre en œuvre un corps central 8 qui est relativement étroit. De préférence, les premier et second moteurs 31, 32 sont parallèles et côte à côte plutôt que l'un derrière l'autre, ce qui permet à la dimension longitudinale ou longueur totale du corps central 8, et donc à la dimension longitudinale ou longueur totale de la caméra 1, d'être suffisamment réduite pour ne pas gêner le passage de la caméra 1 dans un coude situé dans un espace creux. En effet, il est souhaitable que la caméra d'endoscopie présente des dimensions géométriques les plus réduites possibles pour pouvoir s'insinuer au sein des espaces cavitaires plus ou moins étroits et/ou sinueux d'un équipement, par exemple un conduit ou une canalisation présentant un ou plusieurs coude(s) à angle droit et/ou des dimensions radiales relativement faibles.

Une telle configuration permet également d'obtenir de manière avantageuse une caméra d'endoscopie industrielle 1 relativement simple et légère, avec un nombre limité d'éléments, tout en conservant un large éventail de fonctions pratiques, en particulier un champ de vision étendu, rendu possible grâce à la caméra 1 permettant une « *double rotation* » du dispositif de prise de vue 15 embarqué. Le caractère léger de la caméra d'endoscopie industrielle 1 rend particulièrement aisée la manipulation de cette dernière par un opérateur, notamment pour réaliser l'inspection visuelle d'un espace creux, en particulier lorsque la caméra 1 est attachée à un organe de manoeuvre 3 de plusieurs mètres de long, et lorsque l'espace creux est particulièrement long, étroit et/ou coudé, par exemple incluant un coude s'élevant à la verticale.

Alternativement, lesdits premier et second actionneurs 9, 28 comprennent et partagent un même moteur unique (non illustré) pour mettre en rotation respectivement lesdits premier et deuxième arbres rotatifs 29, 30. Ledit moteur unique comprend par exemple deux arbres de sortie, formant chacun au moins en partie l'un différent desdits premier et deuxième arbres rotatifs 29, 30. Ledit moteur unique comprend selon un autre exemple un arbre de sortie unique formant notamment au moins en partie ledit premier ou deuxième arbre rotatif 29, 30.

De façon avantageuse, ledit premier moteur 31 et/ou ledit second moteur 32 (de préférence les deux moteurs 31, 32) est/sont positionné(s) sensiblement à l'extérieur dudit support 2, comme cela est illustré aux figures. Ainsi, de manière préférentielle, au moins l'un, et de préférence les deux, desdits premier et second moteurs 31, 32, est (/sont) positionnés principalement, avantageusement en totalité, en dehors du support 2, et même en regard et/ou dans le prolongement de ce dernier. De préférence, ledit premier moteur 31 et/ou ledit second moteur 32 est/sont destinés à être positionné(s) sensiblement à l'opposé dudit organe de manœuvre 3 par rapport audit support 2. Le support 2 n'embarque avantageusement aucun desdits premier et second actionneurs 9, 28, et plus particulièrement le support 2 n'embarque de préférence aucun desdits premier et second moteurs 31, 32. Les premier et second actionneurs 9, 28 ne sont donc de préférence pas logés dans le support 2, ni entourés par ce dernier, mais sont avantageusement disposés dans le prolongement longitudinal dudit support 2. Cela permet de conférer à la caméra 1 une forme particulièrement fuselée et élancée, avec un diamètre minimal permettant une introduction dans des espaces très restreints.

La caméra d'endoscopie industrielle 1 de l'invention est de préférence relativement étroite et d'une longueur raisonnable, et est donc particulièrement adaptée pour passer dans des cavités étroites, par exemple un espace creux à l'intérieur d'un conduit de faible section transversale ou de faible diamètre, et dans des coudes de ces cavités étroites, par exemple une portion incurvée d'un conduit.

Par exemple, pour un coude à 90° (c'est-à-dire à angle droit) d'un conduit de type DN50 (c'est-à-dire présentant un diamètre nominal, extérieur et/ou intérieur de 50 mm), un ensemble endoscopique, de préférence de forme sensiblement cylindrique, destiné à passer dans le coude et comprenant une caméra d'endoscopie industrielle (par exemple cylindrique) et éventuellement une partie non flexible de l'organe de manœuvre 3 (par exemple son connecteur), devra présenter un diamètre maximum théorique de 45 mm, tandis que sa longueur maximal théorique sera de 125 mm, pour que ledit ensemble endoscopique puisse théoriquement être capable de passer le coude. Bien évidemment, lorsque le diamètre de l'ensemble endoscopique est inférieur à 45 mm, sa longueur maximale théorique est de préférence potentiellement supérieure à 125 mm. Or, les caméras connues présentent des dimensions trop importantes, en longueur et/ou en largueur ou diamètre, pour que l'ensemble endoscopique respectif dont elles font partie puisse passer le coude à 90°. Par exemple, les modèles connus de caméras les plus compactes (en particulier les plus étroites) présentent un diamètre de 45 mm, mais ne conviennent pas pour circuler dans le conduit de type DN50, d'une part parce que le coude à 90° comporte des aspérités ou autres obstacles, et d'autre part parce les ensembles endoscopiques formés avec ces modèles connus de caméras présentent une longueur trop importante, notamment de l'ordre de 150 mm. Ainsi, en augmentant sensiblement la compacité latérale ou radiale de l'ensemble endoscopique grâce à l'utilisation d'une caméra d'endoscopie industrielle 1 selon l'invention, il est en particulier possible d'obtenir un diamètre (ou une largeur) de l'ensemble endoscopique (et/ou de la caméra 1) d'environ 20 à 28 mm (voire un peu plus ou un peu moins, par exemple de 18 à 32 mm), ce qui nécessite que l'ensemble endoscopique présente une longueur maximale théorique maximale avantageusement située entre 130 et 160 mm, par exemple environ égale à 140 mm, 150 mm ou 160 mm, pour qu'il puisse théoriquement être capable de passer le coude. La caméra 1 selon l'invention permet en outre avantageusement d'obtenir un ensemble endoscopie d'une longueur relativement faible, c'est-à-dire par exemple égale et/ou inférieure à 150 mm, voire à 140 mm ou à 130 mm, et est donc en particulier adaptée pour passer un coude (notamment à 90°) d'un conduit relativement étroit (notamment de type DN50). L'ensemble endoscopique obtenu avec la caméra d'endoscopie industrielle 1 selon l'invention, présentant par exemple une longueur totale de 150 ou 130 mm et un diamètre de 28 ou 26 mm (de préférence sensiblement équivalent ou égal à celui de la caméra 1 elle-même), est ainsi tout à fait apte à circuler au sein d'un conduit de type DN50 pourvu d'un (ou plusieurs) coude(s) à 90°.

Grâce à une telle configuration, le diamètre de la structure de tête 14 (ou boule de visualisation) de la caméra d'endoscopie industrielle 1 est, de façon avantageuse, relativement réduit, et il se situe avantageusement en deçà de 30 mm, et ce quelle que soit la direction de visualisation de la caméra. En d'autres termes, de manière préférentielle, quelle que soit la rotation effectuée par la structure de tête 14 par rapport au corps central 8 et/ou par ce dernier par rapport au support 2, ledit premier actionneur 9 étant relié à ladite structure de tête 14 pour mettre en rotation cette dernière par rapport audit corps central 8, le diamètre de l'ensemble endoscopique et/ou de la structure de tête 14 reste relativement réduit, et en dessous de 30 mm.

En résumé, la configuration de caméra d'endoscopie industrielle 1 décrite ci-avant (et optionnellement munie des caractéristiques décrites ci-après) permet avantageusement de diminuer l'encombrement radial ou latéral, c'est-à-dire par exemple la largeur et/ou le diamètre (et/ou encore l'épaisseur), de la caméra d'endoscopie industrielle 1, et ce sans augmenter l'encombrement longitudinal de la caméra 1 voire en diminuant ce dernier, de manière à ce que la caméra 1 puisse passer sans encombre les obstacles rencontrés dans un espace creux (en particulier à l'intérieur d'un conduit étroit, d'une machine, etc.), et notamment un ou plusieurs coudes d'un conduit relativement étroit.

De préférence, ledit premier moteur 31 s'étend longitudinalement entre une première et une deuxième extrémité 35, 36 opposées, ledit second moteur 32 s'étendant longitudinalement entre une troisième et une quatrième extrémité 37, 38 opposées, lesdites première et quatrième extrémités 35, 38 étant positionnées en regard de la structure de tête 14, lesdites deuxième et troisième extrémités 36, 37 étant positionnées en regard du support 2, lesdits premier et deuxième arbres rotatifs 29, 30 débouchant respectivement desdites première et troisième extrémités 35, 37. En d'autres termes, de manière avantageuse, les premier et second moteurs 31, 32 sont disposés tête-bêche, et présentent ainsi chacun un arbre de sortie, l'arbre de sortie du premier moteur 31 et l'arbre de sortie du second moteur 32 étant à l'opposé l'un de l'autre et respectivement formés au moins en partie par lesdits premier et deuxième arbres rotatifs 29, 30. En particulier, ledit premier arbre rotatif 29 s'étend sensiblement à partir du premier moteur 31 vers la structure de tête 14 (et/ou vers ladite extrémité primaire 24) et ledit deuxième arbre rotatif 30 s'étend sensiblement à partir du second moteur 32 vers le support 2 (et/ou vers ladite extrémité secondaire 25).

De préférence, ledit premier arbre 29 est rotatif selon un troisième axe de rotation R3, tandis que ledit deuxième arbre 30 est rotatif selon un quatrième axe de rotation R4, lesdits troisième et quatrième axes de rotation R3, R4 étant avantageusement sensiblement parallèles l'un à l'autre ou confondus. De surcroit, le(s)dit(s) troisième et/ou quatrième axe(s) de rotation R3, R4 est/sont préférentiellement parallèle(s) à (ou alternativement confondu(s) avec) ladite première direction d'extension longitudinale L et/ou ledit deuxième axe de rotation R2. Avantageusement, ledit premier arbre rotatif 29 et/ou ledit deuxième arbre rotatif 30 est/sont monté(s) à rotation relativement audit corps central 8, audit support 2 et/ou à ladite structure de tête 14, tandis que les premier et second moteurs 31, 32 sont reliés fixement au corps central 8.

De préférence, comme illustré aux figures 3 à 5, ledit premier actionneur 9 comprend en outre un dispositif primaire de transformation 39 du mouvement de rotation dudit premier arbre rotatif 29 en mouvement de rotation de ladite structure de tête 14 par rapport audit corps central 8 selon ledit premier axe de rotation R1, lequel est perpendiculaire audit troisième axe de rotation R3. De manière avantageuse, lesdites deuxième et troisième directions d'extension longitudinales L2, L3 sont sensiblement parallèles à ou confondues avec, respectivement, les troisième et quatrième axes de rotation R3, R4.

Par exemple, uniquement à titre illustratif et non limitatif, comme illustré aux figures 3 à 5, ledit dispositif primaire de transformation 39 comprend un premier élément d'engrenage 40, lequel est relié audit premier arbre rotatif 29 de préférence de manière fixe, de sorte qu'un déplacement en rotation dudit premier arbre rotatif 29 selon le troisième axe de rotation R3 entraîne un déplacement en rotation dudit premier élément d'engrenage 40 selon le troisième axe de rotation R3 (ou selon un axe de rotation parallèle à ce dernier). Ledit dispositif primaire de transformation 39 comprend en outre, de manière préférentielle, un second élément d'engrenage 41, lequel est avantageusement relié à (et de préférence solidaire de) ladite roue dentée 23, de sorte qu'un déplacement en rotation dudit second élément d'engrenage 41 par exemple selon ledit axe de rotation primaire A entraîne un déplacement en rotation dudit premier élément d'engrenage 40 selon le troisième axe de rotation R3 lui-même préférentiellement perpendiculaire à l'axe de rotation primaire A (ou entraîne un déplacement en rotation dudit premier élément d'engrenage 40 selon un axe de rotation parallèle à ce dernier troisième axe de rotation R3 lui-même préférentiellement perpendiculaire à à l'axe de rotation primaire A).

Lesdits premier et second éléments d'engrenage 40, 41 sont de préférence conçus pour former un système d'engrenage, et sont donc avantageusement complémentaires l'un de l'autre, de façon que, lorsqu'ils sont positionnés en correspondance l'un de l'autre (en particulier au sein dudit corps central 8), une rotation du premier élément d'engrenage 40, par exemple selon le troisième axe de rotation R3, entraîne une rotation du second élément d'engrenage 41, par exemple selon l'axe de rotation primaire A, ledit axe de rotation R3 et ledit axe de rotation primaire A étant préférentiellement sensiblement perpendiculaires.

Par exemple, toujours de manière illustrative et non limitative, comme illustré aux figures 3 à 5, lesdits premier et second éléments d'engrenage 40, 41 présentent chacun des dents (et/ou des évidements). Les dents (ou évidements) du premier élément d'engrenage 40 s'étendent par exemple selon ledit troisième axe de rotation R3 et/ou des directions respectives parallèles à ce dernier, tandis que les dents (ou évidements) dudit axe dudit second élément d'engrenage 41 s'étendent selon ledit axe de rotation primaire A et/ou des directions respectives parallèles à ce dernier, les dents (ou évidements) respectives des premiers et seconds éléments d'engrenage 40, 41 étant donc avantageusement sensiblement perpendiculaires les unes aux autres.

Préférentiellement, comme illustré aux figures 3 à 5, ledit dispositif primaire de transformation 39 comprend un troisième arbre rotatif 42, qui relie ledit second élément d'engrenage 41 et ladite roue dentée primaire 23 et qui est monté à rotation relativement audit corps central 8, de préférence au sein de ce dernier, par exemple selon l'axe de rotation primaire A, ou selon un axe de rotation sensiblement parallèle à ce dernier. Ledit dispositif primaire de transformation 39 comprend en outre par exemple, de manière illustrative et non limitative, comme illustré à la figure 3, au moins un palier 10 avantageusement prévu et/ou monté dans le corps central 8 et conçu pour recevoir une partie extrémale 33 du troisième arbre rotatif 42 (ce dernier présentant avantageusement une forme sensiblement allongée) afin de permettre la rotation de ce dernier relativement audit corps central 8, par exemple une partie extrémale 33 située à l'opposé par rapport au second élément d'engrenage 41. Selon une autre alternative, non illustrée, le dispositif primaire de transformation 39 comprend deux paliers avantageusement prévus et/ou montés dans le corps central 8 et conçus pour recevoir chacun l'une différente de deux parties extrémales du troisième arbre rotatif 42 (ce dernier présentant avantageusement une forme sensiblement allongée) afin de permettre la rotation de ce dernier relativement audit corps central 8, lesdites deux parties extrémales se situant de part et d'autre du second élément d'engrenage 41, de préférence à l'opposé l'une de l'autre. Optionnellement, la caméra d'endoscopie industrielle comprend un système de roulement à bille (non illustré) qui est prévu entre la partie extrémale 33 du troisième arbre rotatif 42 et le palier 10 correspondant prévu dans le corps central 8. De manière avantageuse, le corps central 8 peut également comprendre en son sein, pour assurer son étanchéité, un ou plusieurs joints (non illustrés) disposés à côté et/ou de part et d'autre de ladite roue dentée 23 et/ou autour dudit troisième arbre rotatif 42, lesdits joints étant en particulier des joints toriques entourant une portion du troisième arbre rotatif 42.

Comme on l'a vu, la structure de tête 14 est avantageusement « enserrée » par ledit corps central 8, en particulier entre lesdites première et troisième sous-parties 11, 13. La deuxième sous-partie 12, quant à elle, est de préférence reliée d'une part aux première et troisième sous-parties 11, 13 (par exemple par soudage ou vissage), et d'autre part au support 2. Ladite deuxième sous-partie 12 est, de manière avantageuse, montée à rotation, relativement à ce dernier, selon le deuxième axe de rotation R2.

De préférence, ledit second actionneur 28 comprend en outre un dispositif secondaire de transformation 53 du mouvement de rotation dudit deuxième arbre rotatif 29 (selon le quatrième axe de rotation R4) en mouvement de rotation dudit corps central 8 par rapport audit support 2 selon ledit premier axe de rotation R2, lequel est avantageusement sensiblement parallèle audit quatrième axe de rotation R4 et à distance de ce dernier.

De façon préférentielle, comme illustré aux figures 3, 4 et 6, le dispositif secondaire de transformation 53 comprend une roue dentée secondaire 44 et une roue dentée tertiaire 45, lesdites roues dentées secondaire 44 et tertiaire 45 étant conçues pour former un système engrenage lorsqu'elles sont positionnées en correspondance, en particulier en correspondance au sein du corps central 8. De préférence, ladite roue dentée secondaire 44 est solidaire du deuxième arbre rotatif 30 et/ou venue de matière avec ce dernier, et peut donc avantageusement effectuer une rotation (selon le quatrième axe de rotation R4) relativement audit corps central 8 lorsque ledit second moteur 32 est mis en marche. Avantageusement, ladite roue dentée tertiaire 45 est solidaire du support 2, et notamment montée fixement relativement à ce dernier, plus particulièrement à une partie du support 2 se trouvant au sein corps central 8, c'est-à-dire montée à l'intérieur de ce dernier. De façon avantageuse, ladite roue dentée tertiaire 45 est fixe par rapport audit support 2, et présente un axe central C sensiblement parallèle à ou confondu avec le deuxième axe de rotation R2 et/ou ladite première direction d'extension longitudinale L1, et sensiblement parallèle à et non confondu avec ledit quatrième axe de rotation R4 et/ou ladite troisième direction d'extension longitudinale L3.

Ainsi, selon un exemple de fonctionnement à titre descriptif uniquement et non limitatif, lorsque le second moteur 32 est mis en marche, la roue dentée secondaire 44, liée au deuxième arbre rotatif 30, se déplace en rotation selon le quatrième axe de rotation R4, et va venir interagir avec la roue dentée tertiaire 45 de façon à se déplacer autour d'elle selon un mouvement de rotation ayant comme axe de rotation l'axe central C, à la manière d'un petit cylindre d'engrenage mobile « *roulant»* autour d'un grand cylindre d'engrenage fixe. En d'autres termes, selon cet exemple, ledit deuxième arbre rotatif 30 tourne sur lui-même selon le quatrième axe de rotation R4 et il tourne autour et à distance de l'axe central C (et/ou dudit deuxième axe de rotation R2 et/ou de la première direction d'extension longitudinale L). Ladite roue dentée secondaire 44, liée audit deuxième arbre rotatif 30 et donc audit second moteur 32, va alors entraîner ce dernier en déplacement en rotation autour de l'axe central C. Le second moteur 32 étant de préférence relié de manière fixe au corps central 8, lequel est monté à rotation relativement audit support 2, le corps central 8 va, dans cet exemple, effectuer un déplacement en rotation selon l'axe central C qui forme alors le deuxième axe de rotation R2. En d'autres termes, ledit quatrième axe de rotation R4 et/ou ladite roue dentée secondaire 44 tournent, lorsque le second moteur 32 est mis en marche, autour d'un axe instantané de rotation formé par ledit axe central C, ladite première direction d'extension longitudinale L1 et/ou ledit deuxième axe de rotation R2.

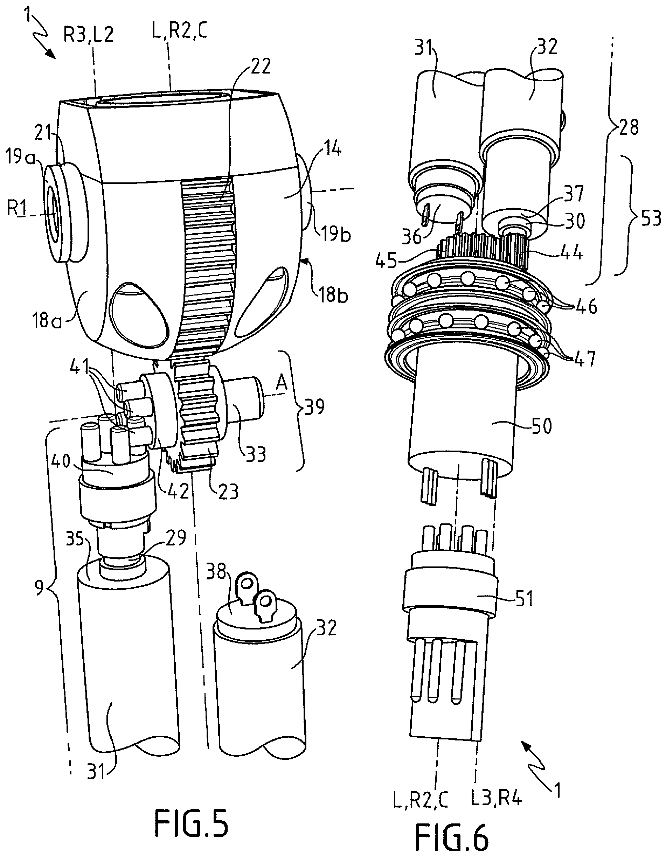

De manière avantageuse, comme illustré aux figures 3 et 6, la caméra d'endoscopie industrielle 1 comprend au moins un voire au moins deux système(s) de roulement à billes 46, 47 disposé(s) à l'interface dudit corps central 8 et dudit support 2, c'est-à-dire sensiblement entre le corps central 8 et le support 2 (ou plus précisément une partie du support 2 positionnée à l'intérieur du corps central 8 et en particulier dans la seconde cavité 27). Chaque système de roulement à billes 46, 47 permet ainsi avantageusement de faciliter la rotation, et plus précisément le guidage en rotation, du corps central 8 relativement audit support 2 selon le deuxième axe de rotation R2. Dans le cas où la caméra d'endoscopie industrielle 1 comprend deux système(s) de roulement à billes 46, 47, assurant ainsi un guidage en rotation plus efficace et plus stable (qu'un seul système de roulement), ceux-ci sont disposés l'un derrière l'autre, c'est-à-dire à une (faible) distance l'un de selon le deuxième axe de rotation R2.

De préférence, la caméra d'endoscopie industrielle 1 comprend également un joint d'interface 48 corps central 8 / support 2, ledit joint d'interface 48 étant plus avantageusement encore sensiblement annulaire et positionné entre ledit corps central 8 et ledit support 2 (plus exactement une partie de support 2 se trouvant à l'intérieur dudit corps central 2), de façon à garantir l'étanchéité du corps central 8. Ledit joint d'interface 48 est par exemple embarqué au sein du corps central 8, plus précisément sensiblement au niveau ou à proximité immédiate de l'extrémité secondaire 25, et présente au moins une forme annulaire au moins partiellement repliée sur elle-même, à la manière d'un joint torique spécifique comprenant une première rondelle en appui sur une surface intérieure du corps central 8 (voire liée à ce dernier), une seconde rondelle en appui sur une surface extérieure du support 2 (et plus précisément sur une surface extérieure de la portion primaire 49, décrite ci-après), et une rondelle intermédiaire faisant le lien entre lesdites première et seconde rondelles. Ledit joint d'interface 48 est ainsi préférentiellement destiné à garantir l'étanchéité entre le support 2 et le corps central 8 lorsque ce dernier est mis en rotation, le joint d'interface 48 « *glissant* » autour du support 2 pendant la rotation du corps central 8.

Avantageusement, certaines des différentes parties de la caméra d'endoscopie industrielle 1, et notamment lesdits support 2, corps centra! 8, et structure de tête 14, comprennent très préférentiellement chacune au moins un boîtier ou des parties de boîtier entourant, étanchéifiant et protégeant au moins partiellement les éléments internes (comme par exemple les premier et second moteurs 31, 32). Lesdits support 2, corps central 8, et structure de tête 14 (et leur boîtiers respectifs) sont en particulier sensiblement rigides et réalisés dans des matériaux appropriés, par exemple de l'acier, notamment pour les boîtiers ou parties de boîtier, des polymères, des matériaux composites, etc.

Préférentiellement, la caméra d'endoscopie industrielle 1 comprend un moyen de gestion de flux électrique, vidéo et/ou de pilotage de la caméra 1, formé par exemple au moins en partie par une carte électronique 43, comme illustré à la figure 3. Le moyen de gestion de flux, et plus précisément la carte électronique 43, est par exemple positionné(e) au sein du corps central 8, et plus précisément au sein de la deuxième sous-partie 12, c'est-à-dire embarqué(e) par ledit corps central 8 et plus précisément par la deuxième sous-partie 12. Le moyen de gestion de flux, et plus précisément la carte électronique 43, est par exemple positionné(e), selon l'exemple illustratif et non limitatif de la figure 3, entre lesdits premier et second moteurs 31, 32, de préférence de façon sensiblement parallèle auxdits deux moteurs 31, 32, ladite carte électronique 43 étant avantageusement de forme sensiblement allongée.

Avantageusement, comme illustré aux figures 3 et 6, le support 2 comprend au moins une portion primaire 49 embarquant au moins en partie un moyen de liaison électrique, vidéo et/ou de pilotage entre le moyen de gestion de flux et un connecteur primaire 51, par exemple formé au moins en partie par un collecteur tournant 50. De préférence, le moyen de liaison comprend également des moyens de connexion du collecteur tournant 50 à un connecteur primaire 51 destinés à assurer l'alimentation du collecteur tournant 50 en flux électrique et/ou de pilotage, et/ou l'alimentation du connecteur primaire 51 en flux vidéo provenant du collecteur tournant 50 (et dont la source trouve son origine dans l'unité de prise de vue 15, par exemple via ladite carte électronique 43). Lesdits moyens de connexion comprennent par exemple des fils électriques (non représentés) notamment embarqués par le support 2.

Ledit connecteur primaire 51 est de préférence destiné à être connecté à l'organe de manœuvre 3 et plus précisément au connecteur de ce dernier, ou alternativement à faire partie de ce dernier.

Comme illustré à la figure 3, le support 2 comprend avantageusement une portion secondaire 52 solidaire de la portion primaire 49, et embarquant plus avantageusement encore ledit connecteur primaire 51.

De manière avantageuse, la portion primaire 49 est emboîtée au moins en partie au sein (c'est-à-dire à l'intérieur) de la seconde portion 50, afin d'assurer la connexion entre le connecteur primaire 51 et le collecteur tournant 50. Ladite roue dentée tertiaire 45 est, de manière préférentielle, solidaire de ladite portion primaire 49 qui lui sert avantageusement de support. Ladite roue dentée tertiaire 45 est par exemple fixée à (et plus précisément sur) ladite portion primaire 49 à l'aide d'une ou plusieurs vis.

Comme illustré à la figure 3, ladite portion secondaire 52 est par exemple reliée de manière fixe à ou venue de matière avec la portion primaire 49). Optionnellement, la portion secondaire 52 amovible, c'est-à-dire détachable de la portion primaire 49, par exemple les portions primaire 49 et secondaire 52 sont vissées l'une à l'autre.

De préférence, la portion secondaire 52 est reliée de manière fixe (et optionnellement amovible, par exemple vissée) avec l'organe de manœuvre 3.

Par exemple, le connecteur (non illustré, mais sensiblement situé au niveau ou à proximité du premier bout 4) de l'organe de manœuvre 3 comprend une partie femelle, en particulier sensiblement creuse, cylindrique et/ou pourvue d'un filetage intérieur, tandis que le support 2 comprend une partie mâle. Cette dernière est en particulier, comme illustré à la figure 3, formée par une surface extérieure de la portion secondaire 52 (ou alternativement de la partie primaire 49) pourvue d'un filetage extérieur 6 complémentaire dudit filetage extérieur, ladite partie femelle étant complémentaire de ladite partie mâle de manière à pouvoir solidariser l'organe de manœuvre 3 (via son connecteur) et ledit support 2 de préférence de manière réversible (par exemple par vissage). Il est ainsi avantageusement possible de coupler ou découpler à volonté ledit organe de manœuvre 3 dudit support 2 et donc de découpler ladite caméra d'endoscopie industrielle 1 dudit organe de manœuvre 3. La caméra d'endoscopie industrielle 1 comprend optionnellement, comme illustré aux figures 1 à 4, un protège-filetage 54 destiné à protéger le filetage extérieur 6 et montable sur ledit support 2 (et plus précisément sur la portion secondaire 52).

## Revendications

1. Caméra d'endoscopie industrielle (1) comprenant au moins :
- un support (2) destiné à être attaché à un organe de manœuvre (3) allongé du genre câble, jonc de poussée, perche, ou gaine souple ;
- un corps central (8) embarquant un premier actionneur (9) ;
- une structure de tête (14) embarquant une unité de prise de vue (15) et montée à rotation relativement audit corps central (8),
ledit premier actionneur (9) étant relié à ladite structure de tête (14) pour mettre en rotation cette dernière par rapport audit corps central (8), le corps central (8) étant monté à rotation relativement audit support (2), **caractérisée en ce que** le corps central (8) embarque en outre un second actionneur (28) destiné à mettre en rotation ledit corps central (8) par rapport audit support (2), ledit premier actionneur (9) et/ou ledit second actionneur (28) étant positionné(s) sensiblement à l'extérieur dudit support (2).

2. Caméra d'endoscopie industrielle (1) selon la revendication précédente, **caractérisée en ce qu'**elle comprend également ledit organe de manœuvre (3) allongé du genre câble, jonc de poussée, perche, ou gaine souple.

3. Caméra d'endoscopie industrielle (1) selon la revendication 1 ou 2, **caractérisée en ce que** ledit support (2) est conçu pour que ledit organe de manœuvre (3) puisse être lui être rattaché sensiblement à l'opposé dudit corps central (8).

4. Caméra d'endoscopie industrielle (1) selon l'une des revendications précédentes, **caractérisée en ce que** ladite structure de tête (14), ledit corps central (8), ledit support (2) et ledit organe de manœuvre (3) sont sensiblement positionnés les uns derrière les autres successivement dans cet ordre.

5. Caméra d'endoscopie industrielle (1) selon l'une des revendications précédentes, **caractérisée en ce que** lesdits premier et second actionneurs (9, 28) comprennent respectivement un premier et un deuxième arbre rotatif (29, 30), lesdits premier et deuxième arbres rotatifs (29, 30) étant sensiblement parallèles.

6. Caméra d'endoscopie industrielle (1) selon la revendication précédente, **caractérisée en ce que** lesdits premier et second actionneurs (9, 28) comprennent respectivement un premier et un second moteur (31, 32) pour mettre en rotation respectivement lesdits premier et deuxième arbres rotatifs (29, 30).

7. Caméra d'endoscopie industrielle (1) selon la revendication précédente, **caractérisée en ce que** ledit premier moteur (31) et/ou ledit second moteur (32) est/sont positionné(s) sensiblement à l'extérieur dudit support (2).

8. Caméra d'endoscopie industrielle (1) selon la revendication 6 ou 7, **caractérisée en ce que** ledit premier moteur (31) et/ou ledit second moteur (32) est/sont destinés à être positionné(s) sensiblement à l'opposé dudit organe de manœuvre (3) par rapport audit support (2).

9. Caméra d'endoscopie industrielle (1) selon l'une des revendications 6 à 8, **caractérisée en ce que** lesdits premier et second moteurs (31, 32) présentent chacun une forme sensiblement allongée et sont disposés sensiblement parallèlement l'un à l'autre au sein du corps central (8).

10. Caméra d'endoscopie industrielle (1) selon la revendication 5 et optionnellement l'une des revendications 6 à 8, **caractérisée en ce que** ledit premier moteur (31) s'étend longitudinalement entre une première et une deuxième extrémité (35, 36) opposées, ledit second moteur (32) s'étendant longitudinalement entre une troisième et une quatrième extrémité (37, 38) opposées, lesdites première et quatrième extrémités (35, 38) étant positionnées en regard de la structure de tête (14), lesdites deuxième et troisième extrémités (36, 37) étant positionnées en regard du support (2), lesdits premier et deuxième arbres rotatifs (29, 30) débouchant respectivement desdites première et troisième extrémités (35, 37).

11. Caméra d'endoscopie industrielle (1) selon l'une des revendications précédentes, **caractérisée en ce que** ladite structure de tête (14) est montée à rotation relativement audit corps central (8) selon un premier axe de rotation (R1), et **en ce que** ledit corps central (8) est monté à rotation relativement audit support (2) selon un deuxième axe de rotation (R2), lequel est sensiblement perpendiculaire audit premier axe de rotation (R1).

12. Caméra d'endoscopie industrielle (1) selon la revendication précédente, l'une des revendications 5 à 10, **caractérisée en ce que** ledit premier arbre (29) est rotatif selon un troisième axe de rotation (R3), ledit premier actionneur (9) comprenant en outre un dispositif primaire de transformation (39) du mouvement de rotation dudit premier arbre rotatif (29) en mouvement de rotation de ladite structure de tête (14) par rapport audit corps central (8) selon ledit premier axe de rotation (R1), lequel est perpendiculaire audit troisième axe de rotation (R3).

13. Caméra d'endoscopie industrielle (1) selon l'une des revendications précédentes, **caractérisée en ce que** ledit corps central (8) présente une forme sensiblement allongée s'étendant longitudinalement entre une extrémité primaire (24) et une extrémité secondaire (25), et comprend, à ladite extrémité primaire (24), une première cavité (26), ladite structure de tête (14) étant logée au moins en partie dans ladite première cavité (26).

14. Caméra d'endoscopie industrielle (1) selon la revendication précédente, **caractérisée en ce que** ledit corps central (8) comprend, à l'extrémité secondaire (25), une seconde cavité (27), ledit support (2) étant logé au moins en partie dans ladite seconde cavité (27).

15. Caméra d'endoscopie industrielle selon la revendication précédente, **caractérisée en ce que** lesdits premier et second actionneurs (9, 28) sont positionnés à l'intérieur du corps central (8) entre lesdites première et seconde cavités (26, 27).

## Patentansprüche

1. Industrielle Endoskopiekamera (1), umfassend mindestens:
- eine Halterung (2), die dazu bestimmt ist, an einem länglichen Manövrierelement (3) der Art eines Kabels, Schubrohrs, Auslegers oder einer flexiblen Hülle angebracht zu werden;
- einen zentralen Körper (8), der einen ersten Aktuator (9) beherbergt;
- eine Kopfstruktur (14), die eine Bildaufnahmeeinheit (15) beherbergt und drehbar relativ zu dem genannten zentralen Körper (8) montiert ist;
wobei der erste Aktuator (9) mit der genannten Kopfstruktur (14) verbunden ist, um diese Letztere in Bezug auf den genannten zentralen Körper (8) in Drehung zu versetzen, wobei der zentrale Körper (8) relativ zu der genannten Halterung (2) drehbar montiert ist, **dadurch gekennzeichnet, dass** der zentrale Körper (8) außerdem einen zweiten Aktuator (28) beherbergt, der dazu bestimmt ist, den genannten zentralen Körper (8) in Bezug auf die genannte Halterung (2) in Drehung zu versetzen, wobei der genannte erste Aktuator (9) und/oder der genannte zweite Aktuator (28) im Wesentlichen außerhalb der genannten Halterung (2) positioniert ist/sind.

2. Industrielle Endoskopiekamera (1) nach dem vorhergehenden Anspruch, **dadurch gekennzeichnet, dass** sie auch das genannte längliche Manövrierelement (3) der Art eines Kabels, Schubrohrs, Auslegers oder einer flexiblen Hülle umfasst.

3. Industrielle Endoskopiekamera (1) nach Anspruch 1 oder 2, **dadurch gekennzeichnet, dass** die genannte Halterung (2) derart ausgelegt ist, dass das genannte Manövrierelement (3) an diesem im Wesentlichen gegenüber dem genannten zentralen Körper (8) angebracht werden kann.

4. Industrielle Endoskopiekamera (1) nach einem der vorhergehenden Ansprüche, **dadurch gekennzeichnet, dass** die genannte Kopfstruktur (14), der genannte zentrale Körper (8), die genannte Halterung (2) und das genannte Manövrierelement (3) im Wesentlichen hintereinander in dieser Reihenfolge positioniert sind.

5. Industrielle Endoskopiekamera (1) nach einem der vorhergehenden Ansprüche, **dadurch gekennzeichnet, dass** der genannte erste und zweite Aktuator (9, 28) jeweils eine erste und eine zweite Drehwelle (29, 30) umfassen, wobei die genannte erste und zweite Drehwelle (29, 30) im Wesentlichen parallel sind.

6. Industrielle Endoskopiekamera (1) nach dem vorhergehenden Anspruch, **dadurch gekennzeichnet, dass** der genannte erste und zweite Aktuator (9, 28) jeweils einen ersten und einen zweiten Motor (31, 32) umfassen, um jeweils die genannte erste und zweite Drehwelle (29, 30) in Drehung zu versetzen.

7. Industrielle Endoskopiekamera (1) nach dem vorhergehenden Anspruch, **dadurch gekennzeichnet, dass** der genannte erste Motor (31) und/oder der genannte zweite Motor (32) im Wesentlichen außerhalb der genannten Halterung (2) positioniert ist/sind.

8. Industrielle Endoskopiekamera (1) nach Anspruch 6 oder 7, **dadurch gekennzeichnet, dass** der genannte erste Motor (31) und/oder der genannte zweite Motor (32) dazu bestimmt ist/sind, im Wesentlichen gegenüber dem genannten Manövrierorgan (3) in Bezug auf die genannte Halterung (2) positioniert zu sein.

9. Industrielle Endoskopiekamera (1) nach einem der Ansprüche 6 bis 8, **dadurch gekennzeichnet, dass** der genannte erste und zweite Motor (31, 32) jeweils eine im Wesentlichen längliche Form aufweisen und im Wesentlichen parallel zueinander im Inneren des zentralen Körpers (8) angeordnet sind.

10. Industrielle Endoskopiekamera (1) nach Anspruch 5 und gegebenenfalls einem der Ansprüche 6 bis 8, **dadurch gekennzeichnet, dass** sich der genannte erste Motor (31) in Längsrichtung zwischen einem ersten und einem zweiten gegenüberliegenden Ende (35, 36) erstreckt, wobei sich der genannte zweite Motor (32) in Längsrichtung zwischen einem dritten und einem vierten gegenüberliegenden Ende (37, 38) erstreckt, wobei das genannte erste und vierte Ende (35, 38) gegenüber der Kopfstruktur (14) positioniert sind, wobei das genannte zweite und dritte Ende (36, 37) gegenüber der Halterung (2) positioniert sind, wobei die genannte erste und zweite Drehwelle (29, 30) jeweils in dem genannten ersten und dritten Ende (35, 37) münden.

11. Industrielle Endoskopiekamera (1) nach einem der vorhergehenden Ansprüche, **dadurch gekennzeichnet, dass** die genannte Kopfstruktur (14) drehbar relativ zu dem genannten zentralen Körper (8) in einer ersten Drehachse (R1) montiert ist, und dadurch, dass der genannte zentrale Körper (8) drehbar relativ zu der genannten Halterung (2) in einer zweiten Drehachse (R2) montiert ist, die im Wesentlichen rechtwinklig zu der genannten ersten Drehachse (R1) ist.

12. Industrielle Endoskopiekamera (1) nach dem vorhergehenden Anspruch, einem der Ansprüche 5 bis 10, **dadurch gekennzeichnet, dass** die genannte erste Welle (29) in einer dritten Drehachse (R3) drehbar ist, wobei der genannte erste Aktuator (9) außerdem eine erste Vorrichtung (39) zur Umwandlung der Drehbewegung der genannten ersten Drehwelle (29) in eine Drehbewegung der genannten Kopfstruktur (14) in Bezug auf den genannten zentralen Körper (8) in der genannten ersten Drehachse (R1) umfasst, die senkrecht zu der genannten dritten Drehachse (R3) ist.

13. Industrielle Endoskopiekamera (1) nach einem der vorhergehenden Ansprüche, **dadurch gekennzeichnet, dass** der genannte zentrale Körper (8) eine im Wesentlichen längliche Form aufweist, die sich in Längsrichtung zwischen einem ersten Ende (24) und einem zweiten Ende (25) erstreckt, und an dem genannten ersten Ende (24) einen ersten Hohlraum (26) umfasst, wobei die genannte Kopfstruktur (14) mindestens teilweise in dem genannten ersten Hohlraum (26) gelagert ist.

14. Industrielle Endoskopiekamera (1) nach dem vorhergehenden Anspruch, **dadurch gekennzeichnet, dass** der genannte zentrale Körper (8) an dem zweiten Ende (25) einen zweiten Hohlraum (27) umfasst, wobei die genannte Halterung (2) mindestens teilweise in dem genannten zweiten Hohlraum (27) gelagert ist.

15. Industrielle Endoskopiekamera nach dem vorhergehenden Anspruch, **dadurch gekennzeichnet, dass** der erste und zweite Aktuator (9, 28) im Inneren des zentralen Körpers (8) zwischen dem genannten ersten und zweiten Hohlraum (26, 27) positioniert sind.

## Claims

1. Industrial endoscopy camera (1), comprising at least:
- a support (2) intended to be attached to an elongate manoeuvring member (3) of the cable, thrust rod, pole or flexible sheath type;
- a central body (8) carrying a first actuator (9);
- a head structure (14) carrying an imaging unit (15) and mounted for rotation in relation to said central body (8),
said first actuator (9) being connected to said head structure (14) in order to rotate the latter with respect to said central body (8), the central body (8) being mounted for rotation in relation to said support (2), **characterised in that** the central body (8) also carries a second actuator (28) intended to rotate said central body (8) with respect to said support (2), said first actuator (9) and/or said second actuator (28) being positioned substantially outside said support (2).

2. Industrial endoscopy camera (1) according to the preceding claim, **characterised in that** it also comprises said elongate manoeuvring member (3) of the cable, thrust rod, pole or flexible sheath type.

3. Industrial endoscopy camera (1) according to claim 1 or 2, **characterised in that** said support (2) is designed so that said manoeuvring member (3) in its case can be attached substantially opposite said central body (8).

4. Industrial endoscopy camera (1) according to one of the preceding claims, **characterised in that** said head structure (14), said central body (8), said support (2) and said manoeuvring member (3) are substantially positioned behind one another successively in this order.

5. Industrial endoscopy camera (1) according to one of the preceding claims, **characterised in that** said first and second actuators (9, 28) comprise respectively a first and second rotary shaft (29, 30), said first and second rotary shafts (29, 30) being substantially parallel.

6. Industrial endoscopy camera (1) according to the preceding claim, **characterised in that** said first and second actuators (9, 28) comprise respectively a first and a second motor (31, 32) for rotating respectively said first and second rotary shafts (29, 30).

7. Industrial endoscopy camera (1) according to the preceding claim, **characterised in that** said first motor (31) and/or said second motor (32) is/are positioned substantially outside said support (2).

8. Industrial endoscopy camera (1) according to claim 6 or 7, **characterised in that** said first motor (31) and/or said second motor (32) is/are intended to be positioned substantially opposite said manoeuvring member (3) with respect to said support (2).

9. Industrial endoscopy camera (1) according to one of claims 6 to 8, **characterised in that** said first and second motors (31, 32) each have a substantially elongate form and are disposed substantially parallel to each other in the central body (8).

10. Industrial endoscopy camera (1) according to claim 5 and optionally one of claims 6 to 8, **characterised in that** said first motor (31) extends longitudinally between first and second opposite ends (35, 36), said second motor (32) extending longitudinally between third and fourth opposite ends (37, 38), said first and fourth ends (35, 38) being positioned facing the head structure (14), said second and third ends (36, 37) being positioned facing the support (2), said first and second rotary shafts (29, 30) emerging respectively from said first and third ends (35, 37).

11. Industrial endoscopy camera (1) according to one of the preceding claims, **characterised in that** said head structure (14) is mounted for rotation in relation to said central body (8) on a first rotation axis (R1), and **in that** said central body (8) is mounted for rotation in relation to said support (2) on a second rotation axis (R2), which is substantially perpendicular to said first rotation axis (R1).

12. Industrial endoscopy camera (1) according to the preceding claim and one of claims 5 to 10, **characterised in that** said first shaft (29) is able to rotate on a third rotation axis (R3), said first actuator (9) further comprising a primary device (39) for transformation of the rotation movement of said first rotary shaft (29) into a rotation movement of said head structure (14) with respect to said central body (8) on said first rotation axis (R1), which is perpendicular to said third rotation axis (R3).

13. Industrial endoscopy camera (1) according to one of the preceding claims, **characterised in that** said central body (8) has a substantially elongate form extending longitudinally between a primary end (24) and a secondary end (25), and comprises, at said primary end (24), a first cavity (26), said head structure (14) being housed at least partly in said first cavity (26).

14. Industrial endoscopy camera (1) according to the preceding claim, **characterised in that** said central body (8) comprises, at the secondary end (25), a second cavity (27), said support (2) being at least partly housed in said second cavity (27).

15. Industrial endoscopy camera according to the preceding claim, **characterised in that** said first and second actuators (9, 28) are positioned inside the central body (8) between said first and second cavities (26, 27).
